# EUROPEAN PATENT SPECIFICATION

(11) **EP 2 517 697 B1**
(45) Date of publication and mention of the grant of the patent: **07.01.2015**
(21) Application number: 10803411.7
(22) Date of filing: 30.11.2010
(51) Int. Cl.: A61K 9/00, A61K 45/06, A61K 31/232, A61K 31/366, A61K 31/40, A61K 31/404, A61K 31/44, A61K 31/505, A61K 31/22, A61K 9/50

(54) **COMBINATION COMPOSITION USEFUL FOR TREATING CARDIOVASCULAR DISEASES**
KOMBINATIONSPRÄPARAT ZUR BEHANDLUNG KARDIOVASKULÄRER ERKRANKUNGEN
COMPOSITION DE COMBINAISON UTILES POUR TRAITER DES MALADIES CARDIOVASCULAIRES

(30) Priority: 23.12.2009 EP 09180660
(43) Date of publication of application: 31.10.2012
(73) Proprietor: Sigma-Tau Industrie Farmaceutiche Riunite SpA, Rom (IT)
(72) Inventor: CAVAZZA, Claudio, I-00186 Rome (IT)
(74) Representative: Ferreira, Maria Silvina
(86) International application number: PCT/PT2010/000053
(87) International publication number: WO 2011/078712

(56) References cited:
- EP-A1- 1 803 440
- WO-A2-2009/059717
- YOKOYAMA M ET AL: "Effects of eicosapentaenoic acid on major coronary events in hypercholesterolaemic patients (JELIS): a randomised open-label, blinded endpoint analysis", LANCET 20070331 GB LNKD- DOI:10.1016/S0140-6736(07)60527-3, vol. 369, no. 9567, 31 March 2007 (2007-03-31), pages 1090-1098, XP002633503, ISSN: 0140-6736
- IRENE SAVELIEVA ET AL: "Primary and secondary prevention of atrial fibrillation with statins and polyunsaturated fatty acids: review of evidence and clinical relevance", NAUNYN-SCHMIEDEBERG'S ARCHIVES OF PHARMACOLOGY, SPRINGER, BERLIN, DE, vol. 381, no. 3, 25 November 2009 (2009-11-25), pages 207-219, XP019798473, ISSN: 1432-1912

## Description

### FIELD OF THE INVENTION

The present invention relates to a combination composition as defined by the claims consisting of a microcapsule suspension, comprising one or more statins in alkyl esters of n-3 PUFA, in which the statins are isolated from contact with the alkyl ester of n-3 PUFA by means of a polymeric membrane that can be easily disintegrated in the gastrointestinal medium, useful for reducing the number of deaths caused by Acute Coronary Syndrome (ACS) and Acute Myocardial Infarction (AMI) and for improving the short- and long-term prognosis in the patients treated.

### BACKGROUND OF THE INVENTION

The microcapsule suspension comprising one or more statins in alkyl esters of n-3 PUFA mentioned above is described in WO 2006/045865.

Acute coronary syndrome (ACS) is a set of signs and symptoms related to the heart. ACS is compatible with a diagnosis of acute myocardial ischemia, but it is not pathognomonic.

The sub-types of acute coronary syndrome include unstable angina (UA, not associated with heart muscle damage), and two forms of myocardial infarction (MI, heart attack), in which the heart muscle is damaged. These types are named according to the findings documented on the electrocardiogram (ECG/EKG) as non-ST segment elevation myocardial infarction (NSTEMI) and ST segment elevation myocardial infarction (STEMI). There can be some variation as to which forms of MI are classified under acute coronary syndrome.

ACS should be distinguished from stable angina, which develops during exertion and resolves at rest. In contrast with stable angina, unstable angina occurs suddenly, often at rest or with minimal exertion, or at lesser degrees of exertion than the individual's previous angina ("crescendo angina"). New onset angina is also considered unstable angina, since it suggests a new problem in a coronary artery.

Though ACS is usually associated with coronary thrombosis, it can also be associated with cocaine use. Cardiac chest pain can also be precipitated by anemia, bradycardia (excessively slow heart rate) or tachycardia (excessively fast heart rate).

Acute myocardial infarction (AMI) causes morphofunctional alterations that often induce progressive left ventricular dilatation ("ventricular remodelling" phenomenon).

Post-AMI ventricular dilatation can be regarded as an overall compensation mechanism aimed at maintaining an adequate cardiac output in the presence of a reduction of the ejection fraction.

The extent of the ventricular dilatation is the most important prognostic indicator in patients with AMI.

Patients with relatively larger ventricular volumes are at greater risk of future cardiac events (Circulation 1987; 76:44-51).

Limiting the ventricular remodelling phenomenon in the post-infarction period is thus of great importance from the clinico-prognostic point of view (Circulation 1994; 89:68-75). Limitation of this phenomenon can be achieved by two mechanisms: (a) by limiting the extent of the infarcted area (which is the main determinant of future dilatation) by means of early myocardial reperfusion (Circulation 1989; 79:441-444) and/or (b) by reducing the parietal stress and consequently the progressive dilatation of the myocardial area not involved in the infarction process by means of the administration of ACE inhibitors.

When the thrombotic obstruction evolves rapidly towards complete, permanent, vascular occlusion, the resulting lack of perfusion gives rise, in the space of a few hours, to myocardial cell necrosis and thus to infarction. The immediate and long-term prognosis will depend upon a series of factors, the most important of which are the size of the necrotic area and the early and late complications resulting from it. It is therefore obvious that the primary aim of modern therapy for acute infarction is to reduce the size of the infarcted area. This objective is achieved with reperfusion procedures, whether pharmacological (thrombolytic agents), mechanical (PTCA), such as angioplasty, or surgical (by-pass). Generally, the earlier and more effective the reperfusion, the smaller will be the necrotic area. The latter is also influenced, albeit to a lesser extent, by other factors, and above all by the consumption of myocardial oxygen, which is conditioned by the heart rate, myocardial contractility and parietal tension. Of fundamental importance, then, will be all those measures, whether pharmacological or otherwise, that reduce cardiac work, while at the same time maintaining an adequate circulatory capacity.

Useful drugs for the treatment of acute myocardial infarction are already known.

Beta-blockers are drugs endowed with antiarrhythmia properties and are significantly more active if used in the early stages of the onset of the infarction.

Nitroderivatives are drugs administered usually by venous infusion and are useful for enhancing myocardial perfusion through the vasodilatation of the epicardial vessels.

Sodium nitroprussiate is a drug that exerts a double action on the arteriolar and venous districts. This compound produces coronary and renal vasodilatation, thus enhancing myocardial perfusion and diuresis.

A given number of patients with acute myocardial infarction continue to die in the first week of hospitalisation and later, even when treated with all appropriate and available pharmacological and technical means. There is therefore a strongly perceived need for the availability of new and known drugs which are useful for reducing the number of deaths due to acute myocardial infarction, where said drugs are used alone or in combination with the normal known drugs which alone would not be capable of saving from death that proportion of patients who die within the first week or later after the onset of in-farction.

The use of PUFA and statins in the cardiological fild is already known.

In WO 02/43659 a combination of statin, docosahexanoic acid, vitamins E, C B6 and B12, folic acid and calcium is described to reduce the risk factors for cardiovascular disease, such as hypercholes-terolaemia and hypertension.

Durrington et al. (see Heart 2001; 85:544-548) describe an omega-3 PUFA concentrate administred to simvastatin treated patients with coronary heart diseases and persisting hypertriglyceridaemia.

US20070191467 discloses a method of use of a combination of an HMG-CoA inhibitor and omega-3 fatty acids for the treatment of patients with hypertriglyceridemia or hypercholesterolemia or mixed dyslipidemia, coronary heart disease (CHD), vascular disease, atherosclerotic disease and related conditions, and for the prevention or reduction of cardiovascular, cardiac, and vascular events.

WO2006/013602 described a combination comprising at least one omega-3 fatty acid, one statin, Coenzyme Q10, resveratrol, one policosanol, pantethine, selenium and zinc. That combination is useful in the treatment of disease form due to insulin resistance and in cardiovascular diseases.

WO2009/059717describes a composition of statin and omega-3 fatty acids used in the treatment of cardiovascular events, in which the omega-3 fatty acids are encapsulated to be separated from the statin.

Yokoama et al. (see Lancet 2007; 369(9567) 1090-1098) disclosed the treatment of patients suffering from several cardiovascular diseases, with a combination of EPA and simvastatin.

IRENE SAVELIEVA ET AL: "Primary and secondary prevention of atrial fibrillation with statins and polyunsaturated fatty acids: review of evidence and clinical relevance", NAUNYN-SCHMIEDEBERG'S ARCHIVES OF PHARMACOLOGY, SPRINGER, BERLIN, DE, vol. 381, no. 3, 25 November 2009 (2009-11-25), pages 207-219, XP019798473:
Primary and secondary prevention of atrial fibrillation with statins and polyunsaturated fatty acids: review of evidence and clinical relevance: a review on possible preventive effects of PUFAs and statins on atrial fibrillation (AF). Statins showed different results on AF depending on pathology, duration of follow-up and history of AF. However no effects on reduction of atrial fibrillation in patients with acute coronary syndromes were found.

### DESCRIPTION OF THE INVENTION

It has now surprisingly been found that the a combination composition consisting of a microcapsule suspension as defined by the claims comprising one or more statins in alkyl esters of n-3 PUFA in which the statins are isolated from contact with the alkyl ester of n-3 PUFA by means of a polymeric membrane that can be easily disintegrated in the gastrointestinal medium, is useful for reducing the number of deaths caused by Acute Myocardial Infarction and Acute Coronary Syndrome, and for improving the short- and long-term prognosis in the treated patients.

Therefore, one object of the present invention is a combination composition as defined by the claims consisting of a microcapsule suspension comprising one or more statins in alkyl esters of n-3 PUFA in which the statins are isolated from contact with the alkyl ester of n-3 PUFA by means of a polymeric membrane that can be easily disintegrated in the gastrointestinal medium, for use for the prevention and/or treatment of acute myocardial infarction and acute coronary syndrome.

In other words the present invention relates to a combination drug product consisting of a microcapsule composed of a polymeric membrane, comprising one or more statins inside, contained within alkyl esters of n-3 PUFA, in which the statins are isolated from contact with the alkyl ester of n-3 PUFA by means of the said polymeric membrane that can be easily disintegrated in the gastrointestinal medium, for use for the prevention and/or treatment of acute myocardial infarction and acute coronary syndrome.

Another object of the present invention is the use of a combination composition consisting of a microcapsule suspension comprising one or more statins in alkyl esters of n-3 PUFA in which the statins are isolated from contact with the alkyl ester of n-3 PUFA by means of a polymeric membrane that can be easily disintegrated in the gastrointestinal medium, for preparing a medicament useful for the prevention and/or treatment of acute myocardial infarction and acute coronary syndrome; for reducing the number of deaths caused by acute myocardial infarction and acute coronary syndrome; for the prevention and/or treatment of atrial fibrillation associated with acute myocardial infarction and acute coronary syndrome; for reducing the day of hospialization due to said atrial fibrillation; for reducing the days of re-hospitalization for any pre-existing or new non cardiovascular comorbidity associated with acute myocardial infarction and acute coronary syndrome; and for improving the short- and long-term prognosis in the treated patients.

The combination according to the invention can also comprise other useful elements, without this substantially impairing the activity.

The medicine according to the invention can be used to treat the individual disease states or to exert a preventive or protective action against them, or to treat a complex pathological picture that includes one or more of the therapeutic aspects seen above.

The combination according to the present invention consists essentially of active ingredients as defined by the claims which are known in the medical sector and already used in clinical practice. Therefore, they are very easy to procure, inasmuch as they are products which have been on the market for some time and are of a grade suitable for human or animal administration.

The statins are a known class of drugs used for lowering cholesterol levels. Statins are available on the market or can be prepared according to known methods described in the literature.

Any statin is suitable for the purposes of the present invention. Examples of statins are simvastatin, lovastatin, fluvastatin, pravastatin, atorvastatin, cerivastatin and rosuvastatin. Among these, the one preferred is simvastatin.

According to the present invention, it is also possible to combine two or more statins, depending on their pharmacological characteristics and on the basis of the common knowledge of experts in the sector.

The term "n-3 PUFA" (also referred to as ω-3 fatty acids or omega-3 fatty acids) relate to a family of long-chain polyunsaturated fatty acids, generally C₁₆-C_{24,} in particular those having a C₂₀-C₂₂ chain, that have in common a carbon-carbon double bond in the n-3 position, i.e. the third bond from the methyl end of the fatty acid. Examples of the most common n-3 fatty acids found in nature are reported in the Table below together with the assigned names.

| **Common name** | **Lipid name** | **Chemical name** |
|---|---|---|
| - | 16:3 (*n*-3) | *all-cis*-7,10,13-hexadecatrienoic acid |
| α-Linolenic acid (ALA) | 18:3 (*n*-3) | *all-cis*-9,12,15-octadecatrienoic acid |
| Stearidonic acid (STD) | 18:4 (*n*-3) | *all-cis*-6,9,12,15-octadecatetraenoic acid |
| Eicosatrienoic acid (ETE) | 20:3 (*n*-3) | *all-cis*-11,14,17-eicosatrienoic acid |
| Eicosatetraenoic acid (ETA) | 20:4 (*n*-3) | *all-cis*-8,11,14,17-eicosatetraenoic acid |
| Eicosapentaenoic acid (EPA) | 20:5 (*n*-3) | *all-cis*-5,8,11,14,17-eicosapentaenoic acid |
| Docosapentaenoic acid (DPA), Clupanodonic acid | 22:5 (*n*-3) | *all-cis*-7,10,13,16,19-docosapentaenoic acid |
| Docosahexaenoic acid (DHA) | 22:6 (*n*-3) | *all-cis*-4,7,10,13,16,19-docosahexaenoic acid |
| Tetracosapentaenoic acid | 24:5 (*n*-3) | *all-cis*-9,12,15,18,21-docosahexaenoic acid |
| Tetracosahexaenoic acid (Nis-inic acid) | 24:6 (*n*-3) | *all-cis*-6,9,12,15,18,21-tetracosenoic acid |

Preferably the n-3 PUFA according to the invention is a mixture of fatty acids having a high content in EPA and DHA, for example with a content in EPA and DHA higher than 25% by weight, preferably from about 30% to about 100% by weight, in particular about between 75% and 95%, and more preferably at least 85% by weight on the total fatty acid weight. Preferably the total content of n-3 PUFA according to the invention is a mixture of fatty acids having at least 90% of n-3 PUFA by weight on the total fatty acid weight.

The term "n-3 PUFA" as used here is intended to encompass their corresponding C₁-C₃ alkyl esters and/or from their salts with pharmaceutically acceptable bases such as sodium hydroxide, lysine, arginine or aminoalcohols such as choline. The ethyl esters are the most widely used and preferred according to the invention.

The most preferred ratio between EPA and DHA is about 0.6-1.1/1.3-1.8; in particular is comprised between 0.9 and 1.5.

Preferably the content of EPA (as ethyl ester) is comprised between 40 and 51% by weight and the content of DHA (as ethyl ester) is comprises between 34 and 45% by weight on the total fatty acids weight.

The omega-3 fatty acids, or their esters or salts, alone or in mixtures thereof, can be procured on the market, or can be prepared by known methods. The mixtures can be specifically formulated for the combination according to the invention.

As already mentioned, the individual components have long been used in human subjects, and therefore their pharmaco-toxicological profiles are known.

This implies that the dosages and ratios of the individual components can be determined by the expert in the sector with normal preclinical and clinical trials, or with the usual considerations regarding the formulation of a dietetic product.

The amounts of the individual compounds advised for the preparation of a pharmaceutical combination composition for human use are the following.
Omega-3 fatty acid: from 500 mg to 2 g/day, preferably 1 g/day;
Simvastatin; from 5 mg to 40 mg/day, preferably 20 mg/day.

The pharmaceutical combination compositions according to the present disclosure are in a unitary form in which the active ingrdients are present in a single pharmaceutical form for oral administration.

As mentioned above, the combination composition of the present invention as defined by the claims is a formulation consisting of a microcapsule suspension of statins in alkyl esters of n-3 PUFA in which the statins are isolated from contact with the alkyl ester of n-3 PUFA by means of a polymeric membrane that can be easily disintegrated in the gastrointestinal medium.

In other words the present invention relates to a combination drug product consisting of a microcapsule composed of a polymeric membrane, comprising one or more statins inside, contained within alkyl esters of n-3 PUFA, in which the statins are isolated from contact with the alkyl ester of n-3 PUFA by means of the said polymeric membrane that can be easily disintegrated disintegrated in the gastrointestinal medium.

This coating provides stabilization of the statin, eliminating the occurrence of degradation products of the statin during the processes of preparing the microcapsule suspension and of incorporating the mentioned microcapsule suspension of statins in alkyl esters of n-3 PUFA in final system of administering the product (soft gelatin capsules, hard gelatin capsules, tablets, granules, etc.), even though these processes are carried out at a temperature exceeding 40 deg. C.

This coating avoids the problems of degradation that statins have when they are formulated in the presence of oils with a high content of alkyl esters of n-3 PUFA.

The pharmaceutical formulation of the present invention is characterized in that it is made up of a suspension comprising an oil with a high content of alkyl esters of polyunsaturated fatty acid (n-3 PUFA) and microcapsules comprising at least one polymer and a statin.

The polymer coating the microcapsules of statins is preferably selected from the group consisting of polyesters, polyacrylates, polycyanoacrylates, polysaccharides, polyethylene glycol, or mixtures thereof. More preferably, the polymer coating the microcapsules of statins is selected from the group consisting of gelatin, carboxymethylcellulose, alginates, carrageenans, pectins, ethyl cellulose hydroxypropyl methylcellulose, cellulose acetophthalate, hydroxypropyl methylcellulose phthalate, methylacrylic acid copolymers (Eudragit L and S), dimethylaminoethylmethacrylate copolymers (Eudragit E), the trimethylammoniumethyl-methacrylate copolymers (Eudragit RL and RS), polymers and copolymers of lactic and glycolic acids or mixtures thereof.

Optionally, a pharmaceutical formulation according to the present invention comprises an antioxidant, preferably vitamin E acetate. According to a preferred embodiment according to the present invention, the pharmaceutical formulation comprises carnitine.

Preferably, the microcapsules represent between 1% and 60% of the total weight of the pharmaceutical formulation according to the present invention, and the amount of statin incorporated in said microcapsules is comprised between 1% and 80% by weight, preferably between 1 and 40% by weight in relation to the total weight of the microcapsules. Preferably, the oil with a high content of alkyl esters of n-3 PUFA has a purity exceeding 60% in alkyl ester of n-3 PUFA.

According to a preferred embodiment according to the present invention, the polymer comprises a plasticizer additive, preferably those plasticizers selected from the group consisting of triethyl citrate, butyl phthalate or mixtures thereof. Other technical additives of the polymer can optionally be incorporated which improve or facilitate the encapsulation process, such as, for example, fluidizing agents, preferably talc.

The ratio between eicosapentaenoic acid (EPA) and docosahexaenoic acid (DHA) is preferably comprised between 0.5 and 2.

According to a preferred embodiment of the present disclosure, the microcapsule suspension is encapsulated by soft gelatin capsules for oral administration. Said soft gelatin capsules preferably have an enteric coating.

The preparation of the microcapsules can be carried out following any of the methods described in the literature. By way of description and without being limited thereto, the different processes of obtaining microcapsules could be grouped into the following categories:

### A) Simple coacervation methods:

A solution of the polymer together with the possible additives of the polymer in a suitable solvent is prepared.

The drug to be encapsulated is suspended in said solution of the polymer and a non-solvent of the polymer is added so as to force the deposit of the polymer on the drug crystals. Examples of these processes can be found in patent documents such as ES 2009346 , EP 0 052 510, or EP 0 346 879.

### B) Complex coacervation method:

This method is based on the interaction between two colloids having opposite electric charges so as to generate an insoluble complex that is deposited on the particles of the drug to be encapsulated, forming a membrane that will isolate the drug. Examples of these processes can be found in patent documents such as GB 1393805.

### C) Double emulsion methods:

The drug to be encapsulated is dissolved in water or in a solution of some other coadyuvant and is emulsified in a solution of the polymer and additives in a suitable solvent, such as for example dichloromethane. The resulting emulsion is in turn emulsified in water or in an aqueous solution of an emulsifying agent, such as polyvinyl alcohol. Once this second emulsion is carried out the solvent in which the polymer and the plasticizer were dissolved in is eliminated by means of evaporation or extraction. The resulting microcapsules are obtained directly by filtration or evaporation. Examples of these processes can also be found in patent documents such as US 4,652,441.

### D) Simple emulsion methods:

The drug to be encapsulated, the polymer and the additives are dissolved together in a suitable solvent. This solution is emulsified in water or in an emulsifier solution, such as polyvinyl alcohol, and the organic solvent is eliminated by evaporation or by extraction. The resulting microcapsules are recovered by filtration or drying. Examples of these processes can also be found in patent documents such as US 5,445,832.

### E) Solvent evaporation methods:

The drug to be encapsulated, the polymer and additives are dissolved together in a suitable solvent. This solution is evaporated and the resulting residue is micronized to the suitable size. Examples of this process can also be found in patent documents such as GB 2,209,937.

The following non limiting examples further illustrate the invention.

### EXAMPLES

### Patients selection

The study population is represented by all patients discharged over a 12-month period (2003-index date) with a primary diagnosis of MI/ACS.

For all patients, a 12-month period preceding the index date (year 2002) is analyzed to identify several cardiovascular and noncardiovascular conditions as documented by hospitalizations or chronic exposures to specific pharmacological treatments, which are used as identifiers of underlying diseases. Briefly, cardiovascular comorbidities include previous presence of hypertension, heart failure (HF), coronary heart disease (CHD), Atrial Fibrillation (AF), diabetes, stroke, transient ischemic attack (TIA), and peripheral vascular disease (PVD). Noncardiovascular conditions include malignancy, chronic obstructive lung disease (COPD), renal insufficiency and depression.

For clinical conditions without a specific prescription pattern (e.g. AF, PVD), only discharge hospital diagnosis was used.

Exposed patients include all these who received over the stated period any n-3 PUFA prescription.

### Identification of sub-cohorts

The study cohort will be analyzed also via stratification and classification related to:
▪ Demographic variables: sex, age (<45, 45-54, 55-64, 65-74, >75 years);
▪ Clinical variables: the presence of specific comorbidity (diabetes, COPD...);
▪ Therapeutic variables: exposure to n-3 PUFA, statin or their combination after the index hospitalization.

This analysis allow to evaluate serious and expected adverse events, occurring in the identified cohort.

### Follow-up

Follow-up for each patient is extended from the index date to 3 years or until the occurrence within 3 years of the following events:
- mortality for any cause;
- re-hospitalization for AMI/ACS (Outcome 1);
- hospitalization for AF (Outcome 2);
- re-hospitalization for any pre-existing or new non cardiovascular (CV) comorbidity (Outcome 3).

The total number of re-hospitalizations for AMI and for any reason is also considered.

The evaluation and modification of the pharmacological treatments, during follow up, with a specific focus on the duration of compliance with n-3 PUFA and/or statins, was monitored in order also to assess the overall acceptability of this drugs, among the many recommended treatments, not only for post-MI/ACS patients, but also for comorbidities.

### Sample size calculation

Administrative databases from different Local Health Authorities (LHAs) were pooled by the Coordinating Center (Laboratory of Phar-macoepidemiology, Consorzio "Mario Negri Sud"), which was responsible for the management and quality control of data.

From an exploratory evaluation of the population covered by the LHAs, considering a sample of 10 LHAs, it is expected that up to 30000 incident hospitalizations for MI/ACS could be included in the observation, corresponding to 5000 patients hospitalized for MI (500 for each LHA), per each of the years of the study (2003-2006).

Such populations should allow:
a) to evaluate comprehensively the short and long term pharmaco-epidemiological profiles of patients discharged with AMI;
b) to describe the prescriptive trend over time of n-3 PUFA, taking into account also the complexity of the patients in terms of comorbidities and related co-therapies and the occurrence of events during the period of observation;
c) to analyze the effects of treatments on cardiovascular outcome. The results obtained are reported in the following Tables 1-3.

**TABLE 1**

| Effect of combination of statins and n-3 PUFA vs. statins alone on outcomes (multivariate analysis and propensity score). | | | | | |
|---|---|---|---|---|---|
| | | | | **MULTIVARIATE ANALYSIS** | **PROPENSITY SCORE** |
| **OUTCOME** | **Mean F.up days (±DS)** | **N** | **Label** | **HR (CI 95%)** | **HR (CI 95%)** |
| **ALL CAUSE MORTALITY** | 605.62 (±547,20) | 1,593 | Statins+n-3 PUFA vs Statins | 0.55 (0.46 - 0.66) p=<0.0001 | 0.56 (0.47 - 0.66) p=<0.0001 |
| **OUTCOME 1 (Acute Myocardial Infarction or Acute Coronary Syndrome)** | 465.32 (±528,61) | 2,230 | Statins+n-3 PUFA vs Statins | 0.62 (0.52 - 0.72) p=<0.0001 | 0.61 (0.52 - 0.72) p=<0.0001 |
| **OUTCOME 2 (hospitalization for Atrial Fibrillation)** | 530.89 (±526,68) | 2,186 | Statins+n-3 PUFA vs Statins | 0.61 (0.50 - 0.70) p=<0.0001 | 0.56 (0.48 - 0.66) p=<0.0001 |
| **OUTCOME 3 (re-hospitalization for any pre-existing or new non cardiovascular comorbidity)** | 195,26 (±340.86) | 6,736 | Statins+n-3 PUFA vs Statins | 0.49 (0.43 - 0.54) p=<0.0001 | 0.35 (0.30 - 0.41) p=<0.0001 |

**TABLE 2**

| Effect of statins, and combination of statins and n-3 PUFA vs. no statins and no n-3 PUFA on outcomes (patients exposed to only n-3 PUFA were eliminated) in the subgroup of patients with previous hospitalizations for Myocardial Infarction and Acute Coronary Syndrome. | | | |
|---|---|---|---|
| | | | **MULTIVARIATE ANALYSIS** |
| **OUTCOME** | **N** | **LABEL** | **HR (CI 95%)** |
| **ALL CAUSE MORTALITY** | 645 | Statins vs No statins and no n-3 PUFA | 0.49 (0.40 - 0.59) p=<0.0001 |
| | | Statins+n-3PUFA vs No statins and no n-3 PUFA | 0.26 (0.19 - 0.35) p=<0.0001 |
| **OUTCOME 1** | 848 | Statins vs No statins and no n-3 PUFA | 0.65 (0.55 - 0.78) P=<0.0001 |
| | | Statins+n-3 PUFA vs No statins and no n-3 PUFA | 0.36 (0.28- 0.47) p=<0.0001 |
| **OUTCOME 2** | 902 | Statins vs No statins and no n-3 PUFA | 0.63 (0.53 - 0.74) p=<0.0001 |
| | | Statins+n-3 PUFA vs No statins and no n-3 PUFA | 0.35 (0.27 - 0.45) p=<0.0001 |
| **OUTCOME 3** | 2,681 | Statins vs No statins and no N-3 PUFA | 0.64 (0.57 - 0.72) p=<0.0001 |
| | | Statins+n-3 PUFA vs No statins and no n-3 PUFA | 0.28 (0.23 - 0.34) p=<0.0001 |

| | | | |
|---|---|---|---|
| (Outcome 1): re-hospitalization for Acute Myocardial Infarction or Acute Coronary Syndrome; (Outcome 2): hospitalization for Atrial Fibrillation; (Outcome 3): re-hospitalization for any pre-existing or new non cardiovascular comorbidity. | | | |

**TABLE 3**

| Effect of combination of statins and n-3 PUFA vs. statins alone on outcomes (multivariate analysis and propensity score). | | | |
|---|---|---|---|
| | | | **MULTIVARIATE ANALYSIS** |
| **OUTCOME** | **N** | **Label** | **HR (CI 95%)** |
| **ALL CAUSE MORTALITY** | 645 | Statins+n-3 PUFA vs Statins | 0.53 (0.40 - 0.69) p=<0.0001 |
| **OUTCOME 1** | 848 | Statins+n-3 PUFA vs Statins | 0.55 (0.44 - 0.70) p=<0.0001 |
| **OUTCOME 2** | 902 | Statins+n-3 PUFA vs Statins | 0.56 (0.44 - 0.69) p=<0.0001 |
| **OUTCOME 3** | 2,681 | Statins+n-3 PUFA vs Statins | 0.44 (0,36 - 0.55) p=<0.0001 |

| | | | |
|---|---|---|---|
| (Outcome 1): re-hospitalization for Acute Myocardial Infarction or Acute Coronary Syndrome; (Outcome 2): hospitalization for Atrial Fibrillation; (Outcome 3): re-hospitalization for any pre-existing or new non cardiovascular comorbidity. | | | |

It is evident that the results reported in Tables 1-3 show that:
(A) all cause of mortality; (B) acute myocardial infarction or acute coronary syndrome; (C) hospitalization for atrial fibrillation; and (D) re-hospitalization for any pre-existing or new non cardiovascular comorbidity, were reduced in a statistically significant manner using the combination of the invention.

It is also evident that the use of n-3 PUFA alone would have been less active than the use of a statine alone, for this reason and for ethical reasons the patients enrolled were not treated with n-3 PUFA alone (the number of deaths would have increased).

As mentioned above the present invention relates to a combination composition consisting of a microcapsule suspension comprising one or more statins in alkyl esters of n-3 PUFA, in which the statins are isolated from contact with the alkyl ester of n-3 PUFA by means of a polymeric membrane that can be easily disintegrated in the gastrointestinal medium.

Experimental data (available, but not reported in this application) show that the administration a combination drug product consisting of a microcapsule composed of a polymeric membrane, comprising one or more statins inside, contained within alkyl esters of n-3 PUFA (as explained above) is bioequivalent to the administration of a statin and n-3 PUFA in sequential manner (sequential administration of the two active ingredients).

Therefore, according to the present invention the use of the aforesaid compounds it is meant indifferently either the co-administration, i.e. the substantially concomitant supplementation of a statin and n-3 PUFA, or the sequential administration of the two active ingredients formulated separately.

It is evident to any expert of the art that the substantially concomitant supplementation of a statin and n-3 PUFA in a single pharmaceutical composition is much more preferable than the administration of the two active ingredients in a coordinated manner, in which the two active ingredients are present in two different pharmaceutical compositions.

## Claims

1. A combination composition consisting of a microcapsule suspension comprising one or more statins in alkyl esters of n-3 polyunsaturated fatty acids (PUFA), wherein the n-3 PUFA is a mixture of fatty acids, or their corresponding alkyl esters having a content in eicosapentanoic acid (EPA) and docosahexaenoic acid (DHA) from about 30% to about 100% of the total fatty acid weight, and in which the statins are isolated from contact with the alkyl ester of n-3 PUFA by means of a polymeric membrane that can be easily disintegrated in the gastrointestinal medium, for use in the prevention and/or treatment of atrial fibrillation associated with acute myocardial infarction and acute coronary syndrome.

2. The combination composition for use according to claim 1, in which the statin is selected from the group consisting of simvastatin, lovastatin, fluvastatin, pravastatin, atorvastatin, cerivastatin and rosuvastatin.

3. The combination composition for use according to claim 1, in which the statin is simvastatin.

4. The combination composition for use according to claim 1, optionally comprising other useful elements which do not impair the activity.

5. The combination composition for use according to claim 4, in which the useful element is Vitamin E acetate.

6. The combination composition for use according to claim 1, suitable for the administration of the following dosages:
omega-3 fatty acid: from 500 mg to 2 g/day;
simvastatin: from 5 mg to 40 mg/day.

7. The combination composition for use according to claim 1, for reducing the days of hospitalization due to atrial fibrillation, wherein the atrial fibrillation is associated with acute myocardial infarction and acute coronary syndrome.

8. The combination composition for use according to claim 7, in which the atrial fibrillation is associated with acute myocardial infarction and acute coronary syndrome.

9. The combination composition for use according to claim 8, for reducing the number of deaths due to atrial fibrillation, wherein the atrial fibrillation is associated with acute myocardial infarction and acute coronary syndrome.

10. The combination composition for use according to claim 7, for reducing the days of re-hospitalization for any pre-existing or new non cardiovascular comorbidity associated with atrial fibrillation, wherein the atrial fibrillation is associated with acute myocardial infarction and acute coronary syndrome.

## Patentansprüche

1. Eine Kombinationszusammensetzung bestehend aus einer Mikrokapselsuspension umfassend ein oder mehrere Statine in Alkylestern von n-3-mehrfach ungesättigten Fettsäuren (PUFA), wobei die n-3-PUFA ein Gemisch aus Fettsäuren oder deren entsprechenden Alkylestern sind aufweisend einen Anteil an Eicosapentaensäure (EPA) und Docosahexaensäure (DHA) von etwa 30% bis etwa 100% des Gesamtgewichtes an Fettsäure und wobei die Statine, durch eine Polymermembran, die im gastrointestinalen Medium leicht zersetzt werden kann, gegen den Kontakt mit den n-3-PUFA-Alkylestern isoliert sind, zur Anwendung in der Vorbeugung und/oder der Behandlung von Vorhofflimmern im Zusammenhang mit einem akuten Myokardinfarkt und einem akuten Koronarsyndrom.

2. Die Kombinationszusammensetzung nach Anspruch 1, **dadurch gekennzeichnet, dass** das Statin aus der Gruppe ausgewählt ist, die aus Simvastatin, Lovastatin, Fluvastatin, Pravastatin, Atorvastatin, Cerivastatin und Rosuvastatin besteht.

3. Die Kombinationszusammensetzung nach Anspruch 1, **dadurch gekennzeichnet, dass** das Statin Simvastatin ist.

4. Die Kombinationszusammensetzung nach Anspruch 1, **dadurch gekennzeichnet, dass** wahlweise weitere nützliche Elemente umfasst sind, welche die Aktivität nicht beeinträchtigen.

5. Die Kombinationszusammensetzung nach Anspruch 4, **dadurch gekennzeichnet, dass** das nützliche Element Vitamin-E-acetat ist.

6. Die Kombinationszusammensetzung nach Anspruch 1, **dadurch gekennzeichnet, dass** diese zur Verabreichung von folgenden Dosierungen geeignet ist:
Omega-3-Fettsäuren: von 500 mg bis 2 g/täglich
Simvastatin: von 5 mg bis 40 mg/täglich.

7. Anwendung der Kombinationszusammensetzung nach Anspruch 1, **dadurch gekennzeichnet, dass** diese zur Verkürzung der Hospitalisierungstage wegen Vorhofflimmern dient, wobei das Vorhofflimmern mit einem akuten Myokardinfarkt und einem akuten Koronarsyndrom verbunden ist.

8. Die Anwendung nach Anspruch 7, **dadurch gekennzeichnet, dass** das Vorhofflimmern mit einem akuten Myokardinfarkt und einem akuten Koronarsyndrom verbunden ist.

9. Die Anwendung nach Anspruch 8, **dadurch gekennzeichnet, dass** diese zur Verringerung der Anzahl an Sterbefällen durch Vorhofflimmern dient, wobei das Vorhofflimmern mit einem akuten Myokardinfarkt und einem akuten Koronarsyndrom verbunden ist.

10. Die Anwendung nach Anspruch 7, zur Verkürzung der Rehospitalisierungstage für jede vorher bestehende oder neue nicht kardiovaskulare Komorbidität verbunden mit Vorhofflimmern, wobei das Vorhofflimmern mit einem akuten Myokardinfarkt und einem akuten Koronarsyndrom verbunden ist.

## Revendications

1. Une composition combinée consistant en une suspension de microcapsules comprenant une ou plusieurs statines dans des esters d'alkyle de n-3-acides gras polyinsaturés (PUFA), dans laquelle le n-3-PUFA est un mélange d'acides gras ou de ses esters d'alkyle correspondants présentant une quantité d'acide eicosapenténoïque (EPA) et d'acide docosahexénoïque (DHA) d'environ 30% jusqu'à environ 100% du poids total d'acide gras et dans laquelle les statines sont isolées du contact avec l'ester d'alkyle de n-3-PUFA par une membrane polymère laquelle peut être facilement désintégrée dans le milieu gastro-intestinal pour l'utilisation dans la prévention et/ou le traitement de la fibrillation auriculaire avec l'infarctus aigu du myocarde et le syndrome coronarien aigu.

2. La composition combinée selon la revendication 1, **caractérisée en ce que** la statine est choisie dans le groupe consistant en simvastatine, lovastatine, fluvastatine, pravastatine, atorvastatine, cerivastatine et rosuvastatine.

3. La composition combinée selon la revendication 1, **caractérisée en ce que** la statine est simvastatine.

4. La composition combinée selon la revendication 1, **caractérisée en ce que** facultativement celle-ci comprend d'autres éléments utiles lesquels ne nuisent pas l'activité.

5. La composition combinée selon la revendication 4, **caractérisée en ce que** l'élément utile est l'acétate de vitamine E.

6. La composition combinée selon la revendication 1, **caractérisée en ce que** celle-ci est adéquate pour l'administration des dosages suivantes:
Acide gras d'omega-3: de 500 mg jusqu'à 2 g/jour
Simvastatin: de 5 mg jusqu'à 40 mg/jour

7. Utilisation de la composition combinée selon la revendication 1, **caractérisée en ce que** celle-ci sert pour réduire les jours d'hospitalisation à cause de la fibrillation auriculaire, dans laquelle la fibrillation auriculaire est associée avec l'infarctus aigu du myocarde et avec le syndrome coronarien aigu.

8. L'utilisation selon la revendication 7, **caractérisée en ce que** la fibrillation auriculaire est associée avec l'infarctus aigu du myocarde et avec le syndrome coronarien aigu.

9. L'utilisation selon la revendication 8, **caractérisée en ce que** celle-ci sert pour réduire le numéro de décès à cause de la fibrillation auriculaire, dans laquelle la fibrillation auriculaire est associée avec l'infarctus aigu du myocarde et avec le syndrome coronarien aigu.

10. L'utilisation selon la revendication 7, **caractérisée en ce que** celle-ci sert pour réduire les jours de réhospitalisation à cause de n'importe quelle comorbidité non cardiovasculaire préexistante ou nouvelle associée à la fibrillation auriculaire, dans laquelle la fibrillation auriculaire est associée avec l'infarctus aigu du myocarde et avec le syndrome coronarien aigu.
